# EUROPEAN PATENT SPECIFICATION

(11) **EP 2 219 658 B1**
(45) Date of publication and mention of the grant of the patent: **21.08.2013**
(21) Application number: 08857635.0
(22) Date of filing: 03.12.2008
(51) Int. Cl.: A61K 35/74, A61P 1/00, A61P 31/00

(54) **COMPOSITION BASED ON PROBIOTIC BACTERIA IN ASSOCIATION WITH A PREBIOTIC AND USE THEREOF IN THE PREVENTION AND/OR TREATMENT OF RESPIRATORY PATHOLOGIES AND/OR INFECTIONS AND IN THE IMPROVEMENT OF INTESTINAL FUNCTIONALITY**
AUF PROBIOTISCHEN, MIT EINEM PRÄBIOTIKUM ZUSAMMENHÄNGENDEN BAKTERIEN BASIERENDE ZUSAMMENSETZUNG UND DEREN VERWENDUNG ZUR VORBEUGUNG UND/ODER BEHANDLUNG VON ATEMWEGSERKRANKUNGEN UND/ODER INFEKTIONEN SOWIE ZUR VERBESSERUNG DER DARMTÄTIGKEIT
COMPOSITION À BASE DE BACTÉRIES PROBIOTIQUES EN ASSOCIATION AVEC UN PRÉBIOTIQUE ET SON UTILISATION DANS LA PRÉVENTION ET/OU LE TRAITEMENT DE PATHOLOGIES ET/OU D'INFECTIONS RESPIRATOIRES ET DANS L'AMÉLIORATION DE LA FONCTION INTESTINALE

(30) Priority: 03.12.2007 IT MI20072260
(43) Date of publication of application: 25.08.2010
(73) Proprietor: Probiotical S.p.a., 28100 Novara (NO) (IT)
(72) Inventor: MOGNA, Giovanni, I-28100 Novara (NO) (IT); STROZZI, Gian Paolo, I-28100 Novara (NO) (IT); MOGNA, Luca, I-28100 Novara (NO) (IT)
(74) Representative: HOFFMANN EITLE
(86) International application number: PCT/EP2008/066700
(87) International publication number: WO 2009/071578

(56) References cited:
- EP-A- 1 195 095
- WO-A-2006/054135
- WO-A-2007/125558
- DE-U1- 20 202 562

## Description

The present invention relates to a composition based on probiotic bacteria and a substance having probiotic properties and the use thereof to prevent and/or treat respiratory pathologies and/or infections and simultaneously improve intestinal functionality, which may be compromised by the therapeutic treatments adopted to resolve said pathological conditions.

Said composition may further comprise also one or more pharmacologically active substances.

In the past fifty years, the use of probiotic bacteria in the food industry has assumed increasing importance.

"Probiotics" are by definition live species-specific micro-organisms which, when ingested or applied in sufficient number, are capable of inducing in the consumer specific functional and beneficial effects on the state of health of the host.

If the action of the probiotic micro-organism plays a pharmacologically active role against pathological forms affecting the host, the probiotic micro-organism can be defined by the term "biotherapeutic agent", which attests to its potential as a valid aid to medical therapy.

Considering, therefore, that the beneficial properties of probiotics can be of a general systemic nature and aimed at resolving specific disorders or diseases, the use thereof is of interest in various fields of application, from the food industry to the pharmaceutical industry.

In the pharmaceutical industry, probiotic bacteria are commonly employed, for example, in the prevention and treatment of intestinal pathologies of varying origin and nature.

The possible beneficial action of probiotic bacteria is also a subject of study, for example, in patients affected by type 2 diabetes mellitus, chronic inflammatory and autoimmune diseases, tumoural pathologies and high serum cholesterol levels.

With regard to respiratory pathologies and/or infections, these are normally treated by relying on the administration of antibiotics and/or anti-inflammatory drugs, sometimes in massive doses and for extended periods.

Unfortunately, the side effects caused by the use of these drugs are often bothersome, harmful and debilitating for the body.

Document WO2006/054135 relates to a probiotic bacteria based composition which is used in the prevention and/or treatment of respiratory pathologies and/or infections and in the contemporaneous improvement of the intestinal functionality.

The object of the present invention is to prevent and/or therapeutically treat respiratory pathologies and/or infections of varying origin and nature without provoking undesired side effects, such as those induced by traditional antibiotic and/or anti-inflammatory treatments.

Another object of the present invention is to prevent and/or therapeutically treat respiratory pathologies and/or infections of varying origin and nature, whilst simultaneously improving and/or regularising the intestinal functionality of the body, often compromised by said pathological conditions.

These and yet other objects, which will become apparent from the detailed description that follows, have been achieved by the Applicant, who has unexpectedly found that a composition as claimed in claim 1, that comprises a mixture consisting of suitable probiotic bacteria and a suitable ingredient having prebiotic properties is able to provide an adequate response to the problems highlighted above.

The term "prebiotic" is commonly used to indicate substances or components of the diet (e.g. fibre) that the body is able neither to digest nor absorb and which, upon reaching the intestinal environment, are capable of selectively stimulating the development and activity of the various microbial groups beneficial for an individual's health.

The association of probiotics with prebiotic substances or foods gives rise to compositions commonly indicated by the term "symbiotic".

It is thus an object of the present invention to use a symbiotic composition comprising a mixture consisting of 5 specific probiotic bacteria and a specific prebiotic substance in order to prepare a medicament for the prevention and/or therapeutic treatment of respiratory pathologies and/or infections, as set forth in the appended independent claim.

Another object of the present invention concerns the above-described composition, as set forth in the appended independent claim.

A further object of the present invention concerns a medicament comprising the above-described composition, as set forth in the appended independent claim.

Yet a further object of the present invention concerns a kit for the coordinated administration of said composition in combination with one or more pharmacologically active substances, as set forth in the appended independent claim.

Preferred embodiments of the present invention are described in the appended dependant claims.

The use of probiotic bacteria, possibly associated with prebiotics, in respiratory pathologies is, to the Applicant's knowledge, little documented in the prior art. For example, EP-A-1773361 teaches that a specific mixture consisting of three probiotic bacterial strains (selected from among: *Bifidobacterium lactis* LMG P-21384; *Lactobacillus rhamnosus* DSM 16605; *Lactobacillus plantarum* LMG P-21021; *Lactobacillus plantarum* LMG P-21020; *Lactobacillus plantarum* LMG P-21022 and *Lactobacillus plantarum* LMG P-21023), if necessary in a mixture with at least one prebiotic, is suitable for the above-specified purpose. Particularly to be preferred is a granulate for oral use containing the following probiotic bacterial strains: a) *Bifidobacterium lactis* LMG P-21384; *Lactobacillus rhamnosus* DSM 16605; c) *Lactobacillus plantarum* LMG P-21020; and, additionally, d) scFOS as a prebiotic and e) glucose as an excipient.

The above-mentioned bacterial strains were deposited, respectively, by Anidral S.r.L., Via Pietro Custodi, 12, 28100 Novara (Italy) and Mofin S.r.L., Via Pietro Custodi, 12, 28100 Novara (Italy), with the following deposit institutions:
*Bifidobacterium lactis* LMG P-21384 (BCCM LMG - Belgian Coordinated Collections of Micro-organisms, Universiteit Gent, on 31 January 2002; depositor Anidral S.r.L.);
*Lactobacillus rhamnosus* DSM 16605 (DSMZ - Deutsche Sammlung von Mikroorganismen und Zellkulturen GmbH, Braunsweig-Germany, on 20 July 2004; depositor Anidral S.r.L.);
*Lactobacillus plantarum* LMG P-21021 (BCCM LMG - Belgian Coordinated Collections of Micro-organisms, Universiteit Gent, on 16 October 2001, depositor Mofin S.r.L.);
*Lactobacillus plantarum* LMG P-21020 (BCCM LMG - Belgian Coordinated Collections of Micro-organisms, Universiteit Gent, on 16 October 2001, depositor Mofin S.r.L.);
*Lactobacillus plantarum* LMG P-21022 (BCCM LMG - Belgian Coordinated Collections of Micro-organisms, Universiteit Gent, on 16 October 2001, depositor Mofin S.r.L.);
*Lactobacillus plantarum* LMG P-21023 (BCCM LMG - Belgian Coordinated Collections of Micro-organisms, Universiteit Gent, on 16 October 2001, depositor Mofin S.r.L.).

Unlike and as compared to the teaching of the prior art, the Applicant has wholly unexpectedly found that the composition of the present invention, described hereinbelow and in the appended independent claim, shows much greater effectiveness in the prevention and/or therapeutic treatment of respiratory pathologies, possibly with simultaneous improvement and/or regularisation of the intestinal functionality of the body. Furthermore, said composition has revealed to be particularly useful for preparing an influenza vaccine.

The symbiotic composition according to the present invention comprises (as its active ingredient):
a) a mixture comprising the following bacterial strains (probiotic component):
   - *Lactobacillus plantarum* LMG P-21020;
   - *Lactobacillus plantarum* LMG P-21021;
   - *Lactobacillus rhamnosus* DSM 16605;
   - *Lactobacillus rhamnosus* DSM 19739;
   - *Bifidobacterium lactis* LMG P-21384;
   and
b) a prebiotic component comprising at least one scFOS, i.e. short-chain fructo-oligosaccharide, or Gos, galacto-oligosaccharides for use as claimed in claim 1.

According to the invention said mixture comprises all five bacteria strains or consists of all five bacteria strains.

Said probiotic bacterial strains were deposited, respectively, by Anidral S.r.L., Via Pietro Custodi, 12, 28100 Novara (Italy), and Mofin S.r.L., Via Pietro Custodi, 12, 28100 Novara (Italy), with the following deposit institutions:
*Lactobacillus plantarum* LMG P-21020 (BCCM LMG - Belgian Coordinated Collections of Micro-organisms, Universiteit Gent, on 16 October 2001, depositor Mofin S.r.L.);
*Lactobacillus plantarum* LMG P-21021 (BCCM LMG - Belgian Coordinated Collections of Micro-organisms, Universiteit Gent, on 16 October 2001, depositor Mofin S.r.L.);
*Lactobacillus rhamnosus* DSM 16605 (DSMZ - Deutsche Sammlung von Mikroorganismen und Zellkulturen GmbH, Braunsweig-Germany, on 20 July 2004; depositor Anidral S.r.L.);
*Lactobacillus rhamnosus* DSM 19739 (DSMZ - Deutsche Sammlung von Mikroorganismen und Zellkulturen GmbH, Braunsweig-Germany, on 27 September 2007; depositor Anidral S.r.L.);
*Bifidobacterium lactis* LMG P-21384 (BCCM LMG - Belgian Coordinated Collections of Micro-organisms, Universiteit Gent, on 31 January 2002; depositor Anidral S.r.L.);
Said scFOS, or short-chain fructo-oligosaccharides, consist in a blend of non-digestible glucides, commonly obtained by conversion of sugar from sugar beets and comprising a molecule of sucrose to which, on average, one to three molecules of glucose are bonded.

Said composition further comprises a suitable quantity of excipients, selected according to the method of administration envisaged for the composition itself.

The composition of the invention is preferably formulated in a mixture with appropriate excipients such as inert diluents, vehicles, lubricants, dispersing agents, anti-agglomerants, flavourings, sweeteners, stabilisers, preservatives, antioxidants and additives such as amino acids, vitamins, enzymes, plant extracts and osmotically active agents commonly used in the pharmaceutical formulation art.

Solely by way of non-limiting example, among the particularly preferred excipients and additives mention may be made of maltodextrins, e.g. potato maltodextrin, food grade insoluble fibres, starch, tween, silicon dioxide, talcum, flavourings such as mandarin, grapefruit, orange, lemon, apricot, peach, strawberry, raspberry, bilberry, apple, banana, tutti frutti, yoghurt, sucrose, glucose, fructose, sorbitol, mannitol, xylitol, maltitol, acesulfame, saccharine, aspartame, sucralose, taumatin, ascorbic acid, parabens, glutamine, arginine, superoxide dismutase and glutathione.

Particularly preferred, in the case of oral administration, are the following:
- maltodextrin, preferably potato maltodextrin, as an inert diluent;
- insoluble fibre (food grade), as an anti-agglomerant;
- silicon dioxide, as an anti-agglomerant.

Particularly preferred embodiments of the present invention are compositions for oral administration.

Typical preferred formulation forms are, for example, capsules, beads, ready-to-drink solutions or suspensions, powders or granulates in sachets (to be suspended or dissolved in water or in non-carbonated, non-alcoholic beverages at the time of use) or analogous forms, tablets, effervescent formulations and undercaps containing the powder part of a formulation to be included in bottles containing the liquid part thereof.

The compositions of the present invention can also be formulated in coated, lacquered, encapsulated or microencapsulated form so as to be gastro-resistant.

Said compositions may also be formulated in a controlled-release form, so as to release the active ingredients selectively within the intestinal tract, in particular in the colon.

Among the preferred embodiments of the present invention, mention may be made of those formulations in which the preferred bacterial strains of the invention are preferably used in lyophilised form or freeze-dry.

The lyophilisation of said strains, on their own or in a mixture with suitable excipients, is achieved using techniques and equipment commonly employed in processes for the freeze-drying of pharmaceutical and/or food compositions.

The compositions of the present invention are prepared in a traditional manner, depending on the type of formulation to be produced, using preparation techniques known to the person skilled in the pharmaceutical art. By way of non-limiting example, a granular formulation, to be suspended or dissolved in water at the time of use, will be prepared by intimately mixing the components of the composition (active ingredients, coadjuvants, excipients), reducing them to the desired granulometry and degree of moisture before packaging them in single-dose sealed sachets.

A controlled-release composition, on the other hand, will be prepared for example by microencapsulating or microcoating the microgranulated mixture of the component substances of the formulation with suitable mixtures of biocompatible polymers (e.g. Eudragit polymers of varying type and structure, gum arabic, starch, gelatine, polyvinylpyrrolidone, carboxymethyl cellulose, polyvinyl alcohol, alginates, pectins, polyacrylates, ethyl cellulose, cellulose acetate or nitrate, polyethylene, polypropylene, silicones and nylon) preferably resistant to the gastric juices of the stomach and capable of releasing said components after they have remained for a suitable period of time in the gastrointestinal tract or on exposure to the pH values typical of the colon. The microencapsulated mixture thus obtained will be used, for example, for the preparation of tablets, capsules or beads, according to the type of commercial presentation chosen.

### Example 1: preferred composition of the invention

As an example of a preferred embodiment of the invention, which is not intended to limit the scope of the invention itself, a composition to be administered to adult patients, packaged (in the traditional manner, as described above) in sachets of granules will now be described.

The minimum declared count, to be guaranteed throughout the entire shelf life of the product, is 5 billion CFU/probiotic species. The overdosage at time zero (initial count) has been determined based on the required shelf life of the product, which must have a stability of two years. The overdosage factor, depending on the physiochemical characteristics of the formulation and the intrinsic properties of the probiotic strains, preferably used in lyophilised form, is generally in the range of I to 30 times.

Considering that the strains used possess a count of 150 bln/gram, the quantity of product present in an individual sachet will range from 6.499 to 3.599 grams.

Table 1 below shows the detailed composition of the present embodiment:

**Table 1: composition of a sachet for oral administration to adults**

| **Composition** | mg per sachet | Initial count per sachet | Declared count per sachet | Function |
|---|---|---|---|---|
| | | | | |
| *L. plantarum* LMG P- 21020 | from 500 to 16.7 | from 150 to 5 bln | 5 bln | |
| *L. plantarum* LMG P- 21021 | from 500 to 16.7 | | | Active ingredients: |
| *L. rhamnosus* DSM 16605 | from 500 to 16.7 | from 150 to 5 bln | 5 bln | probiotic component |
| *L. rhamnosus* DSM 19739 | from 500 to 16.7 | | | |
| *B. lactis* LMG P-21384 | from 1,000 to 33.4 | from 150 to 5 bln | 5 bln | |
| Fructo-oligosaccharides | 3,000 | | | Active ingredients: |
| (FOS) | | | | prebiotic component |
| Potato maltodextrin | 270 | | | Excipients: |
| | | | | inert diluent |
| Insoluble dietary fibre | 132 | | | Excipients: |
| Silicon dioxide | 97 | | | anti-agglomerants |
| ***TOTAL*** | **from 6.499 to 3.599** | | | |

| | | |
|---|---|---|
| Bacterial counts: | LMG P-21020 & LMG P-21021 | = 150 bln/gram |
| | DSM 16605 & DSM 19739 | = 150 bin/gram |
| | LMG P-21384 | = 150 bln/gram |

In a more preferred embodiment of the invention, the overdosage factor is between 2 and 15 times; particularly preferred, between 3 and 7 times.

### Example 2: particularly preferred composition of the invention

In a particularly preferred example of embodiment, the above-described overdosage factor is equal to 5 times: each probiotic species is thus added to the product at time zero in a quantity of 25 billion CFU per sachet.

The composition intended for administration to adult patients is packaged (in the traditional manner, as described above) in sachets of granules containing the ingredients shown in Table 2 below, in a quantity of 4.0 grams per sachet.

**Table 2: composition of a sachet for oral administration to adults**

| **Composition** | mg per sachet | % by weight | Initial count per sachet | Declared count per sachet | Function |
|---|---|---|---|---|---|
| | | | | | |
| *L. plantarum* LMG P- 21020 | 83.5 | 2.09 | 25bln | 5 bln | Active ingredients: |
| *L. plantarum* LMG P- 21021 | 83.5 | 2.09 | | | probiotic component |
| *L. rhamnosus* DSM 16605 | 83.5 | 2.09 | | | |
| *L. rhamnosus* DSM 19739 | 83.5 | 2.09 | 25 bln | 5 bln | |
| *B. lactis* LMG P-21384 | 167 | 4.17 | 25 bln | 5 bln | |
| | | | | | Active ingredients: |
| Fructo-oligosaccharides | 3,000 | 75.00 | | | prebiotic |
| (FOS) | | | | | component |
| Potato maltodextrin | 270 | 6.75 | | | Excipients: |
| | | | | | inert diluent |
| Insoluble dietary fibre | 132 | 3.30 | | | Excipients: |
| | | | | | anti-agglomerants |
| Silicon dioxide | 97 | 2.42 | | | |
| TOTAL | 4,000 | 100.00 | | | |

| | | |
|---|---|---|
| Bacterial counts: | LMG P-21020 & LMG P-21021 | = 150 bln/gram |
| | DSM 16605 & DSM 19739 | = 150 bln/gram |
| | LMG P-21384 | = 150 bln/gram |

The envisaged dose is one sachet per day; the contents must first be dissolved/suspended in water or another non-carbonated beverage at room temperature and preferably taken on an empty stomach to allow rapid transit of the preparation through the gastro-duodenal tract.

As a rule, a treatment of approximately three months is recommended, preferably to be started with the approaching of the cold and flu season.

In another particularly preferred embodiment, the composition of the present invention further comprises at least one pharmacologically active substance, intended to perform an action in combination with that provided by said active ingredients.

Advantageously, such associations have revealed to be synergetic, thus making it possible to employ relatively low doses of the pharmaceutical active ingredient, and hence significantly decrease the possible side effects induced by administration of the drug on its own.

Preferred pharmacologically active substances are selected, for example, from among: antibiotics, anti-inflammatory, immunomodulating, mucolytic and spasmolytic agents and vitamins.

Said pharmacologically active substances can be suitably formulated in a mixture with the other components of the composition, so that they may be taken together in a single administration.

Said pharmacologically active substances can also be formulated and packaged separately in order to permit an independent administration (also at different times if necessary) of the components, albeit in such a manner as to maintain the synergetic effect thereof, according to the patient's needs.

In this case, independent packages are prepared, containing, respectively, the composition of the present invention and the pharmacologically active substance or substances.

The separate packages as described above are then inserted in a special kit to enable the patient to take them in sequence, or separately, so as to benefit from a therapy that is suitably coordinated in relation to his or her needs.

Solely by way of example, a kit like the one described above may contain a number of sachets, or capsules, for the oral administration of the compositions of the present invention, in combination with a suitable number of doses of antibiotic and/or a multi-vitamin complex and/or a mucolytic drug, sufficient for a week of therapy.

The composition of the present invention has demonstrated to be particularly useful, preferably, for the prevention and and/or treatment of the following pathologies:
1. influenza-like syndromes, often characterised by fever and illnesses affecting the respiratory system (commonly indicated in the sector by means of the abbreviation ILI, i.e. *Influenza Like Illnesses);*
2. bronchitic pathologies of varying nature (including chronic ones);
3. pathologies affecting the upper respiratory tract, e.g. laryngitis and tracheitis (commonly indicated in the sector by means of the abbreviation URTI, i.e. *Upper Respiratory Tract Infections*);
4. common cold;
5.cough.

By way of non-limiting example, to provide supporting evidence of the broad applicative potential of the present invention, an illustration is given below of the results of a clinical study conducted with the preferred composition thereof, as described previously.

A randomised double-blind placebo-controlled prospective study was conducted to investigate the effectiveness of the symbiotic composition of Example 2, described above, in maintaining intestinal homeostasis and, at the same time, its protective potential against infectious pathologies affecting the respiratory tract during the winter season, i.e. its capacity to improve the protection of the body and re-establish a normal state of health versus respiratory infections.

250 subjects with comparable characteristics in terms of age, occupation and lifestyle were enrolled in the study.

84 of said patients were treated with the composition of Example 1 (Sample 3).

Another 84 patients forming a control group were treated with the same composition of Example 1, in which, however, the prebiotic FOS had been replaced with a different prebiotic (GOS, i.e. galacto-oligosaccharides) (Sample 1).

82 patients were administered a placebo consisting of 3.5 grams of potato maltodextrin (Sample 2). The mean age of the 3 different samples was respectively 42 years (standard deviation 15.3 years), 45 years (standard deviation 16.3 years) and 42 years (standard deviation 19.5 years).

The composition was prescribed in the granular form in single-dose sachets, to be taken orally after dissolving the contents in a non-alcoholic, non-carbonated beverage every morning for 90 consecutive days.

The trend in the state of health of the subjects enrolled in the study was monitored by having them fill out a daily diary in which they recorded the presence, on a day-to-day basis, of respiratory system pathologies (coryza, coughing, laryngitis and tonsillitis, febrile inflammation of the upper breathing passages, bronchitis and pneumonia), specifying the duration thereof, the subjective perception of symptoms, the perceived severity and any therapy undertaken.

The data were collected in a database, which enabled a classification of the characteristics of each single episode.

To compare the frequency of events among groups, the chi-square test with Yates' correction was used, whereas the duration and severity of the episodes (understood as independent events) observed among the groups were compared using ANOVA (ANalysis Of VAriance), except in the case of non-homogeneity of variances (as determined by Bartlett's test), where it was replaced by the Kruskall-Wallis test. The program "Epi Info version 6.04d" was used for the statistical analysis.

The results obtained with respect to the change in intestinal functionality demonstrated a statistically significant improvement in intestinal motility in the two groups treated with the symbiotic compositions versus the placebo; a certain preference was also observed for the treatment with the symbiotic composition of Example 2, according to the present invention.

The effect of reducing the duration of ARIs (Acute Respiratory Infections) also showed to be significant (4.59 for Sample 3; 4.71 for Sample 1; 6.10 for the placebo group), with a significant difference for total ARIs, colds and coughs. In this case as well the group treated with the symbiotic composition of Example 1, according to the present invention (Sample 3), responded better to the treatment.

Substantially to the same degree, significant differences were observed in the number of days per person for which the subjects affected by respiratory pathologies were absent from work.

Moreover, no differences were observed among the various groups in the use of pharmacological therapy.

A comparison with an analogous study conducted on a number of patients who were administered the preferred composition of patent application EP-A-1773361, mentioned earlier, unexpectedly showed that the symbiotic composition of the present invention is significantly more active both when used for preventive and for therapeutic purposes against respiratory pathologies and/or infections.

These findings demonstrate that a regular, long-term intake of the symbiotic composition according to the present invention can have a marked positive impact on the body's health, both from a preventive and therapeutic standpoint, with respect to respiratory pathologies and/or infections.

With regard in particular to prevention, it may be affirmed that regular intake of said symbiotic composition can protect the body more effectively against the occurrence of the above-specified pathologies than a traditional influenza vaccine, without inducing the negative symptoms associated with the latter (e.g. general malaise, occasional intermittent fever, bone and joint pain and allergic reactions).

Consequently, the symbiotic composition of the present invention can also be used for the preparation of a medicament that acts like an influenza vaccine.

In addition, it has also been observed that the intake of a symbiotic composition according to the present invention is simultaneously able to improve and/or regularise the body's intestinal functionality, often compromised by said pathologies.

Accordingly, the present invention is also concerned with the symbiotic composition described previously and in the appended claims, with particular reference to the composition described above in Example 2.

Furthermore, another object of the present invention is a new bacterial strain *Lactobacillus rhamnosus -* DSM 19739, identified and described in the previous description, which is one of the essential components of the symbiotic composition of the present invention.

Said strain was isolated from human faecal samples using methods known to persons skilled in the art. Optimal growth of the strain is achieved, according to methods commonly employed in the art, in MRS culture broth (DIFCO, ref. 288130) at 37° C.

The strain has the following characteristics: it is rod shaped; it shows good growth also at temperatures of 15°C and 45°C; it does not produce spores; it is gram positive; it is optionally heterofermentative; and it produces L isomer of lactic acid.

Another object of the present invention is a Kit comprising at least two containers.

In a first container a symbiotic composition, according to the present invention, is contained (first component).

In a second container, at least one pharmacologically active substance (second component). The above containers are separately packaged for the independent administration, sequential or not sequential of said components.

In a further and preferred embodiment of the present invention, the symbiotic composition consists of:
a) a mixture consisting of the above mentioned five bacterial strains (as a probiotic component); and
b) scFOS (as a prebiotic component).

In the symbiotic composition, the probiotic component and the prebiotic component are comprised in a weights ration from 1:10 to 10:1, preferably from 1:5 to 5:1, more preferably from 1:3 to 3:1.

In a preferred embodiment, the weights ration from 1:2 to 2:1 for example 1:1.

## Claims

1. A symbiotic composition for use in the prevention and/or therapeutic treatment of respiratory pathologies and/or infections with simultaneous improvement and/or regularisation of the intestinal functionality comprising:
a) a mixture comprising the following bacterial strains:
- *Lactobacillus plantarum* LMG P-21020;
- *Lactobacillus plantarum* LMG P-21021;
- *Lactobacillus rhamnosus* DSM 16605;
- *Lactobacillus rhamnosus* DSM 19739;
- *Bifidobacterium lactis* LMG P-21384;
and
b) a prebiotic component comprising at least a scFOS, short-chain fructo-oligosaccharide, or GOS, galacto-oligosaccharides.

2. The composition for use according to claim 1, for preparing an influenza vaccine.

3. The composition for use according to any of the preceding claims, wherein said symbiotic composition further comprises a quantity of excipients, selected according to the method of administration envisaged for the composition itself.

4. The composition for use according to claim 3, wherein said excipients are the following:
- potato maltodextrin;
- insoluble dietary fibre;
- silicon dioxide.

5. The composition for use according to any of the preceding claims, wherein said composition further comprises at least one pharmacologically active substance.

6. The composition for use according to claim 5, wherein said pharmacologically active substance is present directly in a mixture with the other components of said composition or is formulated and packaged separately therefrom.

## Patentansprüche

1. Symbiotische Zusammensetzung zur Verwendung in der Vorbeugung und/oder therapeutischen Behandlung von Atemwegserkrankungen und/oder -infektionen mit gleichzeitiger Verbesserung und/oder Regularisierung der Darmfunktionalität umfassend
(a) eine Mischung umfassend die folgenden Bakterienstämme:
- *Lactobacillus plantarum* LMG P-21020;
- *Lactobacillus plantarum* LMG P-21021;
- *Lactobacillus rhamnosus* DSM 16605;
- *Lactobacillus rhamnosus* DSM 19739;
- *Bifidobacterium lactis* LMG P-21384;
und
(b) eine präbiotische Komponente umfassend wenigstens ein scFOS, kurzkettiges Fructo-Oligosaccharid, oder GOS, Galacto-Oligosaccharide.

2. Zusammensetzung zur Verwendung gemäß Anspruch 1 zur Herstellung eines Influenza-Impfstoffs.

3. Zusammensetzung zur Verwendung gemäß einem der vorstehenden Ansprüche, worin die symbiotische Zusammensetzung ferner eine Menge Excipienten ausgewählt gemäß dem Verabreichungsverahren, das für die Zusammensetzung selbst vorgesehen ist.

4. Zusammensetzung zur Verwendung gemäß Anspruch 3, worin die Excipienten die folgenden sind:
- Kartoffel-Maltodextrin;
- unlösliche Ballaststoffe;
- Siliziumdioxid.

5. Zusammensetzung zur Verwendung gemäß einem der vorstehenden Ansprüche, worin die Zusammensetzung ferner wenigstens eine pharmakologisch aktive Substanz umfasst.

6. Zusammensetzung zur Verwendung gemäß Anspruch 5, worin die pharmakologisch aktive Substanz direkt in einer Mischung mit den anderen Komponenten der Zusammensetzung vorliegt oder separat davon formuliert und verpackt ist.

## Revendications

1. Composition symbiotique destinée à être utilisée dans la prévention et/ou le traitement thérapeutique des pathologies respiratoires et/ou des infections avec amélioration et/ou régularisation simultanée de la fonctionnalité intestinale comprenant :
a) un mélange comprenant les souches bactériennes suivantes :
- *Lactobacillus plantarum* LMG P-21020 ;
- *Lactobacillus plantarum* LMG P-21021 ;
- *Lactobacillus rhamnosus* DSM 16605 ;
- *Lactobacillus rhamnosus* DSM 19739 ;
- *Bifidobacterium lactis* LMG P-21384 ;
et
b) un composant prébiotique comprenant au moins un scFOS, fructo-oligosaccharide à courte chaîne, ou des GOS, galacto-oligosaccharides.

2. Composition destinée à être utilisée selon la revendication 1 pour préparer un vaccin antigrippal.

3. Composition destinée à être utilisée selon l'une quelconque des revendications précédentes où ladite composition symbiotique comprend en outre une certaine quantité d'excipients, choisis selon le procédé d'administration envisagé pour la composition elle-même.

4. Composition destinée à être utilisée selon la revendication 3 où lesdits excipients sont les suivants :
- de la maltodextrine de pomme de terre ;
- des fibres alimentaires insolubles ;
- du dioxyde de silicium.

5. Composition destinée à être utilisée selon l'une quelconque des revendications précédentes où ladite composition comprend en outre au moins une substance pharmacologiquement active.

6. Composition destinée à être utilisée selon la revendication 5 où ladite substance pharmacologiquement active est présente directement dans un mélange avec les autres composants de ladite composition ou est formulée et conditionnée séparément de ceux-ci.
